# EUROPÄISCHE PATENTANMELDUNG

(11) **EP 1 348 757 A1**
(43) Veröffentlichungstag der Anmeldung: **01.10.2003**
(21) Anmeldenummer: 02016793.8
(22) Anmeldetag: 26.07.2002
(51) Int. Cl.: C12M 1/34, G01N 33/487, C12Q 1/00

(54) **Vorrichtung und Verfahren zur Detektion von zellulären Vorgängen mittels Lumineszenzmessungen**

(30) Priorität: 27.03.2002 EP 02006978
(71) Anmelder: Micronas GmbH, 79108 Freiburg (DE); Klapproth, Holger, Dr., 79108 Freiburg (DE); Micronas Holding GmbH, 79108 Freiburg (DE)
(72) Erfinder: Lehmann, Mirko, 79102 Freiburg (DE); Klapproth, Holger, Dr., 79108 Freiburg (DE)
(74) Vertreter: Bickel, Michael

(57) **Zusammenfassung**

Die vorliegende Erfindung betrifft eine Vorrichtung zur Detektion eines Lumineszenzereignisses in, an oder in der unmittelbaren Umgebung einer Zelle, eines Zellverbandes oder eines Gewebes, die folgende Merkmale aufweist:
(a) ein Trägerelement (1) mit einer für das direkte oder indirekte Ankoppeln von Zellen präparierten Oberfläche (100),
(b) wenigstens einen optischen Detektor (2) zum Empfangen eines Lumineszenzsignals, der in dem Trägerelement (1) unterhalb der Oberfläche (100) integriert ist,
gekennzeichnet, durch folgende weitere Merkmale:
(c) eine die Oberfläche (100) unter Bildung eines Hohlraumes (70) überdeckende Abdeckung (7), die eine Zuflussöffnung (8) und eine Abflussöffnung (9) aufweist,
(d) eine an die Zuflussöffnung (8) angeschlossene Anregungsquelle (21).

## Beschreibung

Die vorliegende Erfindung betrifft ein Verfahren und eine Vorrichtung zur ortsspezifischen Detektion eines Lumineszenzereignisses, bzw. zur Detektion eines Lumineszenzsignals in, an oder in der unmittelbaren Umgebung einer zur analysierenden Zelle.

Eine gattungsgemäße Vorrichtung zur optischen Untersuchung einer Zelle ist aus der EP 0 881 490 A2 bekannt. Diese Vorrichtung umfasst ein Substrat, insbesondere ein Halbleitersubstrat, mit einer für die Aufnahme von Zellen geeigneten Oberfläche, einer Anzahl von unterhalb der Oberfläche ausgebildeten Photodetektoren sowie mehreren oberhalb der Oberfläche angeordneten Lichtquellen. Die matrixartig angeordneten Detektoren erfassen das durch die Lichtquellen ausgesendete Licht, das bedingt durch eine auf die Oberfläche aufgebrachte Zelle an verschiedenen Detektoren unterschiedliche Lichtintensitäten hervorruft, aus welchen eine Information über die Geometrie der Zelle gewonnen werden kann.

Für einige Anwendungen, beispielsweise in der Pharmaforschung, ist es besonders relevant, das Verhalten von Zellen bei einer chemischen oder biochemischen Anregung untersuchen zu können. Vor allem die Untersuchung von Stoffwechselvorgängen an lebenden Zellen ist von besonderem Interesse, da damit z.B. der Einfluss eines neuen potentiellen Medikamentes beobachtet werden kann. Eine häufig durchgeführte Messung ist hierbei die intrazelluläre Kalziumbestimmung mittels kalziumsensitiver Farbstoffe, z.B. Fura II. Aus A.L. Miller, E. Karplus, L.F. Jaffe: "Coeneterate Imaging [Ca2+]ᵢ with Aequorin Using a Photon Imaging Detector.", Meth Cell Biol 40, 305 (1994), ist es bekannt, Zellen, beispielsweise mittels osmotischer Schockbehandlung, mit einem biolumieszenten Stoff, beispielsweise Aqueorin, zu beladen. Intrazelluläre Kalziumsignale als Antwort auf chemische Reize werden dabei durch die Biolumineszenz des Aequorin sichtbar gemacht.

Ziel der vorliegenden Erfindung ist es, eine kompakte und einfach realisierbar Vorrichtung und ein Verfahren zur Detektion von zellulären Vorgängen mittels Lumineszenzmessungen bei chemischen oder biochemischen Anregungen zur Verfügung zu stellen.

Dieses Ziel wird durch eine Vorrichtung gemäß den Merkmalen des Anspruchs 1 und ein Verfahren gemäß der Merkmale des Anspruchs 16 gelöst. Vorteilhafte Ausgestaltungen der Erfindung sind Gegenstand der Unteransprüche.

Die erfindungsgemäße Vorrichtung umfasst ein Trägerelement mit einer für das direkte oder indirekte Ankoppeln von Zellen präparierten Oberfläche, wenigstens einen optischen Detektor zum Empfangen eines Lumineszenzsignals, der in dem Trägerelement unterhalb der Oberfläche integriert ist, eine die Oberfläche unter Bildung eines Hohlraumes überdeckende Abdeckung, die eine Zuflussöffnung und eine Abflussöffnung aufweist, sowie eine an die Zuflussöffnung angeschlossene Anregungsquelle.

Die Anregungsquelle bildet ein Reservoir für eine chemische oder biologische Substanz, die beispielsweise den Stoffwechsel der Zelle beeinflusst, wobei die Stoffwechselvorgänge durch Lumineszenz sichtbar gemacht und mittels des wenigstens einen Detektors detektiert werden.

Unter dem Begriff "Lumineszenz" werden im folgenden sämtliche, durch ein von der Anregungsquelle abgegebenes Medium hervorgerufenen Lichtemissionen, im weiteren Sinne auch die Aussendung von ultravioletter und infraroter Strahlung zusammengefasst, die nicht durch hohe Temperaturen, sondern durch vorangegangene biologische oder chemische Anregung verursacht wird. Die Lumineszenz zeigenden Stoffe werden Luminophore genannt. Wie dem Fachmann bekannt ist, kann eine solche Lumineszenz hervorgerufen werden durch chemische Anregung, die dann als Chemolumineszenz oder Biolumineszenz bezeichnet ist. Dieser Prozess unterliegt den allgemeinen Grundsätzen der Quantenmechanik und bewirkt eine Anregung der Atome und Moleküle, die anschließend unter Emission von Licht, welches erfindungsgemäß detektiert wird, in den Grundzustand zurückkehren.

Die erfindungsgemäße Vorrichtung, die zur chemischen Anregung der Zelle zur Detektion der ausgesendeten Lumineszenzsignale dient, umfasst bei einer Ausführungsform ein Wellenlängenfilter zwischen der Oberfläche und dem wenigstens einen Detektor. Vorzugsweise sind eine Vielzahl von Detektoren vorhanden, wobei den einzelnen Detektoren Wellenlängenfilter mit unterschiedlichen Durchlasscharakteristiken zugeordnet sein können, um selektiv Lumineszenzsignale unterschiedlicher Wellenlängen detektieren zu können.

Das Trägerelement ist bei einer Ausführungsform als Halbleiterkörper ausgebildet, wobei eine an die Detektoren angeschlossene Auswerteschaltung vorzugsweise in dem Halbleiterkörper integriert ist. Die Verwendung eines anorganischen Halbleitermaterials, wie beispielsweise Silizium, besitzt den Vorteil, dass herkömmliche, hinlänglich bekannte Technologieprozesse, beispielsweise CMOS-Prozesse zur Herstellung des Trägers mit den Detektoren und der Auswertschaltung eingesetzt werden können.

Die Integration der Auswerteschaltung in dem Halbleiterchip ermöglicht in unmittelbarer Nähe zu der zu analysierenden Zelle eine Vorverarbeitung der Detektorssignale. Somit handelt es sich bei dieser bevorzugten Ausführungsform der vorliegenden Erfindung um eine "intelligente" Sensoreinrichtung, die wesentlich mehr leistet, als rein passive Sensoren. Beispielsweise können die Ausgangssignale der elektrooptischen Sensoren durch eine mitintegrierte Schaltung so aufbereitet werden, dass sie über Ausgangsschaltungen und Anschlusskontakte relativ problemlos nach außen geführt werden können. Ferner kann die Vorverarbeitung aus der Digitalisierung der analogen Sensorsignale und ihre Umwandlung in einen geeigneten Datenstrom bestehen. Des weiteren kann das signal-tonoise ratio (Signal-Rausch-Verhältnis) durch die in der erfindungsgemäßen Vorrichtung verwirklichten Nähe des Detektors zum Ort der Signalverarbeitung aufgrund kurzer Signalwege sehr stark verbessert werden. Darüber hinaus sind auch weitere Verarbeitungsschritte möglich, mit denen z.B. die Datenmenge reduziert werden kann oder die der externen Verarbeitung und Darstellung dienen. Damit ist es möglich, dass die verbleibende Auswertung der optischen Signale und ihre Darstellung über einen Personal Computer (PC) erfolgen kann. Ferner kann die erfindungsgemäße Vorrichtung so ausgestaltet sein, dass die vorzugsweise verdichteten bzw. aufbereiteten Daten über Infrarot- oder Funkverbindung an entsprechend ausgestattete Empfangsstationen übermittelt werden können.

Darüber hinaus besteht auch die insbesondere nach Kostengesichtspunkten interessante Möglichkeit, organische Halbleitermaterialien, die beispielsweise in der EP-A-1085319 beschrieben sind, für den Träger mit den Detektoren einzusetzen.

Die Detektoren, die unterhalb der für die Aufnahme von Zellen präparierten Oberfläche angeordnet sind, sind vorzugsweise als Photodioden, CCD-Sensoren oder Photoleiter ausgebildet. Vorzugsweise sind mehrere Detektoren matrixartig in dem Träger integriert, um so eine räumlich aufgelöste Lumineszenzmessung durchführen zu können.

Bei einer Ausführungsform ist vorgesehen, dass die an den Zufluss der Abdeckung angeschlossene Anregungsquelle angesteuert durch die Auswerteschaltung ein Anregungsmedium an die Zuflussöffnung, welchem die an der Oberfläche immobilisierte Zelle ausgesetzt wird. Zur Steuerung der Medienzufuhr ist beispielsweise ein Ventil in einer Zuführleitung zwischen der Anregungsquelle und der Zuflussöffnung angeordnet, das durch die Ansteuerschaltung angesteuert ist.

Um die Zellen an die Oberfläche des Trägerelements anzubinden besteht die Möglichkeit, eine Adhäsionsmatrix oder ein die Zelladhärenz und/oder das Zellwachstum förderndes Medium, beispielsweise Gelatine, auf die Oberfläche aufzubringen und die Zellen bereits auf diesem Nährmedium wachsen zu lassen. Außerdem besteht die Möglichkeit Zellen, auf eine die Zellen immobilisierende Schicht, beispielsweise negativ geladenes Polystyrol, auf die Oberfläche aufzubringen.

Bei einer Ausführungsform der Erfindung ist vorgesehen, dass bereits herstellerseitig eine Zelle oder ein Zellverband an der Oberfläche immobilisiert ist, wodurch es einem Kunden ermöglicht wird unmittelbar Messungen an der Zelle nach Zugabe eines von ihm gewählten Anregungsmediums in die Anregungsquelle durchzuführen.

Vorzugsweise umfasst das Trägerelement mehrere Detektoren oder Detektorenfelder, die räumlich voneinander getrennt sind, um so parallel Messungen an mehreren Zellen oder Zellverbänden durchführen zu können. Diese einzelnen Detektoren oder Detektorfelder sind vorzugsweise derart getrennt angeordnet, dass im wesentlichen keine Lichtemission eines Punktes oder Feldes von dem oder den Detektoren eines anderen Punktes oder Feldes empfangen werden kann. So können die einzelnen Detektionsorte in jeweiligen Vertiefungen angeordnet sein, wie sie zum Beispiel von üblichen Mikrotiterplatten bekannt sind. Bevorzugt sind erfindungsgemäß muldenartige Vertiefungen und solche, deren seitlichen Wandungen im wesentlichen senkrecht zur Oberfläche des Sensorchips angeordnet sind. Die jeweiligen Abmessungen einer solchen Vertiefung kann der Fachmann in Kenntnis des Anwendungsbereichs frei wählen, wobei die Vertiefung vorzugsweise um wenigstens 100nm in die Oberfläche des Trägerelements eingesenkt ist.

Alternativ können auf der im wesentlichen planaren Oberfläche senkrecht nach oben gerichtete Trennmittel angeordnet sein, deren Abmessungen vom Fachmann in Kenntnis des gewünschten Anwendungsbereiches und der räumlichen Abmessung der Zellen ausgewählt werden können. Die Anbringung entsprechend geeigneter Trennmittel kann beispielsweise durch anodisches Bonden oder durch sogenannte Flip-Chip- Verfahren erfolgen. Eine solche Vorrichtung mit mehreren Detektorfeldern ermöglicht erfindungsgemäß ein sensorgestütztes elektrooptisches Bildaufnahmeverfahren.

Bei einem erfindungsgemäßen Verfahren zur Detektion von zellulären Vorgängen mittels Detektion von Lumineszenzereignisses in, an oder in der unmittelbaren Umgebung einer Zelle, eines Zellverbandes oder eines Gewebes, ist vorgesehen, eine Vorrichtung bereitzustellen, die ein Trägerelement mit einer für das direkte oder indirekte Ankoppeln von Zellen präparierten Oberfläche, wenigstens einen in dem Trägerelement unterhalb der Oberfläche integrierten optischen Detektor zum Empfangen eines Lumineszenzsignals, eine die Oberfläche unter Bildung eines Hohlraumes überdeckende Abdeckung mit einer Zuflussöffnung und einer Abflussöffnung und eine an die Zuflussöffnung angeschlossene Anregungsquelle aufweist. An der für die Aufnahme von Zellen präparierten Oberfläche wird wenigstens eine Zelle immobilisiert, wobei anschließend ein physikalisches oder chemisches Anregungsmedium aus der Anregungsquelle in den Hohlraum zugeführt und daraus resultierende Lumineszenzereignisse mittels des wenigstens einen Detektors erfasst werden.

Die Lumineszenzereignisse werden vorzugsweise zeitlich aufgelöst erfasst. Das heißt, nach der Abgabe eines Anregungsmediums an den Umgebung der Zelle, werden die Lichtverhältnisse an den Detektoren zu mehreren aufeinanderfolgenden Zeitpunkten, beispielsweise im Takt von, 1ns ausgewertet.

Um Stoffwechselvorgänge in der Zelle mittels Lumineszenz sichtbar zu machen, ist der Einsatz von Luminophoren erforderlich, die auf die interessierenden Stoffwechselprodukte mit Lumineszenz reagieren, die durch die Detektoren erfasst wird.

Für den jeweiligen Anwendungsfall geeignete Luminophore können von einem Fachmann werden abhängig von dem zu detektierenden Stoffwechselprodukt, mit welchem die Luminophore reagieren sollen, in hinlänglich bekannter Weise ausgewählt werden.

Man kann Luminophore mit unterschiedlichen Halbwertzeiten unterscheiden. Die Halbwertzeit gibt an, wie lange Lumineszenz nach einer Anregung messbar ist. Die Auswahl eines Luminophors mit einer für den jeweiligen Anwendungsfall geeigneten Halbwertzeit liegt im Ermessen des Fachmanns. Besonders geeignet sind insbesondere Luminophore, deren Halbwertzeit deutlich größer als 5ns, vorzugsweise zwischen 100µs und 2000µs, liegt.

Grundsätzlich geeignet sind organische Luminophore, Selten Erden Metalle (SEE) bzw. Lanthaniden oder Actinidenverbindungen, "Microspheres" (z.B. FluoSpheres® Europium Luminescent Microspheres, Molecular Probes) oder Nanokristalle von Halbleitern, wobei letztere neben ihren Lumineszenzeigenschaften insbesondere eine relativ kleine Größe (wenige nm) und eine hohe Stabilität (kein Photobleaching) aufweisen. Weitere geeignete Luminophore sind Erdalkalihalogenide mit Gitterfehlstellen, wie sie z.B. durch Dotierungen (Fremdionen) oder radioaktive Strahlung hergestellt werden können.

Die meisten vorgenanten Farbstoffe sind nur für die Messung an oder in der unmittelbaren Umgebung der Zellen geeignet.

Allerdings gibt es Verfahren um Zellmembranen für Farbstoffe und Reportermoleküle permeabel zu machen. Derartige Verfahren sind z.B.:
- Acetoxymethyl (AM) ester loading
- Acid loading (insbesondere für Pfanzenzellen)
- ATP-induzierte Ppermeabilisierung
- Cationic liposome delivery
- Electroporation
- Hypoosmotischer Shock
- Influx pinocytic cell-loading reagent

Ein geeignetes Beladesystem ist z.B. in K. Barber, et al.: "Delivery of Membrane-Impermeant Fluorescent Probes into Living Neural Cell Populations by Lipotransfer.", Neurosci Lett 15 207, 17 (1996) beschrieben.

Bei einer Ausführungsform des Verfahrens ist vorgesehen, die durch den oder die Detektor(en) detektierten Lumineszenzsignals mit einem Referenzwert zu vergleichen. Dieser Referenzwert kann in einem Speicher in dem Halbleiterkörper gespeichert werden und kann beispielsweise durch eine Messung vor der Anregung der Zelle erhalten werden. Der Referenzwert kann auch parallel zu der Messung an der Zelle erhalten werden, indem mehrere räumlich getrennte Detektoren oder Detekorfelder bereitgestellt werden, wobei im Bereich eines Detektors oder Detektorfeldes keine Zelle immobilisiert ist, so dass detektierte Lumineszenzsignale dieses Detektors oder Detektorfeldes als Referenzwert verwendet wird, der Licht bzw. Strahlungseinflüsse berücksichtigt, die nicht durch die zu analysierende Zelle bedingt sind, wie z.B. die Eigenfluoreszenz von Systemkomponenten, und die somit herausgerechnet werden können.

Sofern die Sensoroberfläche das Design einer Microarraynordnung aufweist, bei der eine Vielzahl von Detektorfeldern mit jeweils einer Anzahl von Detektoren vorhanden sind, kann die Detektion der Messfeld- bzw -punktsignalwerte sequentiell erfolgen, indem z.B. ganze Zeilen oder Spalten der Sensoroberfläche bzw Teile derselben nacheinander detektiert werden (Multiplexanwendung).

Die Verwendung mehrerer paralleler Detektorfelder, an denen jeweils dieselben Messungen durchgeführt werden, bietet darüber hinaus den Vorteil, dass mehrere Messergebnisse vorliegen, aus denen das Gesamt-Messergebnis gemittelt werden kann.

Die Ausgangssignale der Detektoren können in dem Halbleiterchip ausgewertet oder mittels geeigneter Schaltungseinrichtungen nach einer Analog-Digitalumsetzung einer externen Auswerteeinrichtung zugeführt werden.

Für den Fachmann ist klar, dass die Wahl des Detektors bzw. des Materials von der zu detektierenden Emissionswellenlänge des Farbstoffes abhängt. Grundsätzlich besitzt der Detektor aufgrund des sogenannten "Halbleiterbandgaps" je nach Materialwahl (z.B. Silizium oder Germanium) unterschiedliche Empfindlichkeiten bezüglich Wellenlänge der detektierten Lumineszenzsignals. Im bevorzugten Falle der Verwendung einer Silizium-Photodiode wird ein Empfindlichkeitsbereich geschaffen, der vom infraroten bis in das ultraviolette Wellenspektrum reicht, wobei die Empfindlichkeit zwischen diesen Bereichen am größten. (s. z.B. B. Streetman: "Solid State Electronic Devices", , Prentice-Hall, Inc., ISBN 0-13-10 436379-5, S. 201-227 (1995).

Um Lumineszenzen unterschiedlicher Wellenlängen detektieren zu können werden entweder wellenlängenspezifische Photoelemente oder aber herkömmliche Photodioden ausgewählt, die mit aufgelegten, aufgebrachten, aufgedampften oder integrierten Wellenlängenfiltern ausgestattet sind. So ist z.B. bekannt, dass Siliziumnitrid im Gegensatz zu Siliziumoxid UV-Licht nicht durchläst, und dass Polysilizium UV-Strahlung absorbiert (s. z.B. V.P. Iordanov et al.: "Integrated high rejection filter for NADH fluorescence measurements", Sensors 2001 Proceedings, Vol. 1,8 -10 Mai, S. 106- 111, AMA-Service (2001)). Daher kann auf die Gateoxidschicht im Rahmen des üblichen CMOS-Prozesses Nitrid oder Polysilizium deponiert werden, wodurch auf der Photodiode entsprechende Filter geschaffen werden. So hat z.B. NADH (Nicotinamid Adenine Dinucleotid) eine Anregungswellenlänge von 350 nm und eine Emissionswellenlänge von 450 nm. Durch Aufbringung eines Filters, der 350nm ausfiltert, kann daher die Sensitivität erhöht werden.

Dieser Effekt kann genutzt werden, um bei paralleler Verwendung von z.B. zwei unterschiedlichen Luminophoren, von denen beispielsweise nur einer Licht im UV-Bereich emittiert, eine differentielle Detektion zu ermöglichen, da die hierfür vorgesehenen Detektoren UV-sensitiv ausgestaltet sind oder nicht. Ferner bietet dieser Effekt die Möglichkeit, gegebenenfalls störende Eigenfluoreszenzen anwesender Materialien mit bekannter Emissionswellenlänge durch Bereitstellung entsprechender Filter aus dem Messverfahren herauszunehmen. Ein Beispiel hierfür ist die parallele Verwendung von Europium-Chelaten (Emission bei ca. 620 nm) und mit Kupfer dotiertem Zinksulfid (Emission bei ca. 525 nm), die durch hinreichend voneinander verschiedenen Emissionswellenlängenbereichen eine Zweifarbdetektion ermöglichen, z.B. innerhalb eines Bereichs eines Detektorpunktes bzw .-feldes, indem z.B. die eine Hälfte der Sensoren eines Detektorpunktes bzw. -feldes mit einem Tiefpassfilter und die andere Hälfte der Sensoren des gleichen Punktes oder Feldes mit einem Hochpassfilter ausgestattet ist.

Zusätzlich oder alternativ können unterschiedliche Luminophoren parallel eingesetzt werden, sofern ihre physikalischen bzw. optischen Eigenschaften hinreichend voneinander abweichen. Beispielsweise werden erfindungsgemäß die unterschiedlichen Anregungswellenlängen zweier zu verwendender Luminophore A und B und/oder deren unterschiedlichen Halbwertzeiten genutzt. Dies kann z.B. durch Bereitstellung von zwei unterschiedlich dotierten Nanokristallen erfolgen.

Die Signale der Detektoren werden durch eine Auswertungseinheit aufgenommen. Die Auswertungseinheit besitzt einen sehr schnellen Konverter zur Umwandlung analoger Detektorsignale in digitale Werte, die gespeichert werden. Eine Auswertung der digitalen Werte wird vorzugsweise in Echtzeit vorgenommen, kann jedoch auch zeitlich verzögert erfolgen. Zur Auswertung der digitalen Werte kann ein gewöhnlicher Mikroprozessor verwendet werden. Für den Fall, dass das Lumineszenzsignal für eine eindeutige Detektion zu schwach ist, kann im Rahmen einer bevorzugten Ausführungsform der Detektion eine Erhöhung der Nachweissensitivität über eine Integration mehrerer Einzelmessungen erreicht werden. Dabei erfolgt eine identische Messung mehrfach und die Messergebnisse werden aufaddiert. Dies kann sowohl direkt auf dem Sensorchip als auch nach der Messung über geeignete Software erfolgen.

Die vorliegende Erfindung wird nachfolgend in Ausführungsbeispielen anhand von Figuren näher erläutert. In den Figuren zeigt:
- Figur 1: ein erstes Ausführungsbeispiel einer erfindungsgemäßen Vorrichtung zur Detektion von zellulären Vorgängen mittels Lumineszenzmessungen in Seitensicht im Querschnitt,
- Figur 2: ein zweites Ausführungsbeispiel einer erfindungsgemäßen Vorrichtung in Seitenansicht im Querschnitt,
- Figur 3: ein drittes Ausführungsbeispiel einer erfindungsgemäßen Vorrichtung in Seitenansicht im Querschnitt,
- Figur 4: ein viertes Ausführungsbeispiel einer erfindungsgemäßen Vorrichtung in Seitenansicht im Querschnitt.

In den Figuren bezeichnen sofern nicht anders angegeben gleiche Bezugszeichen gleiche Teile mit gleicher Bedeutung.

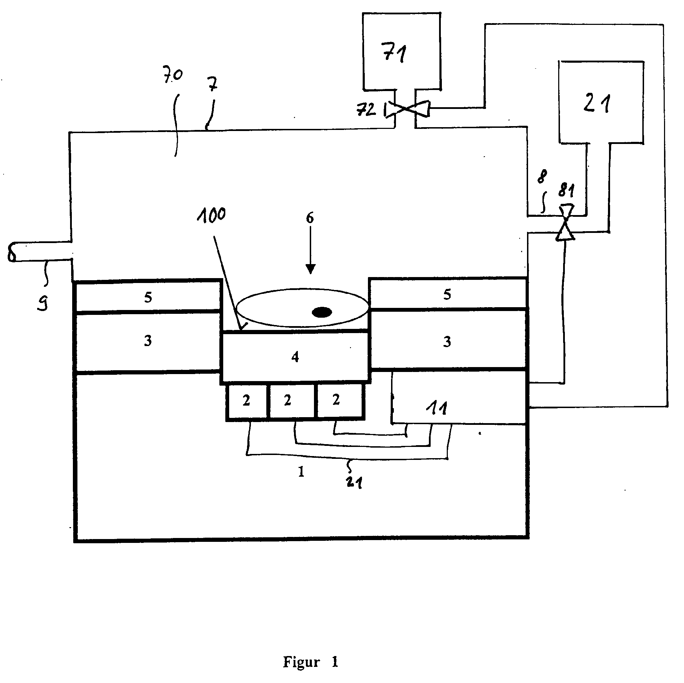

Figur 1 zeigt ein erstes Ausführungsbeispiel einer erfindungsgemäßen Vorrichtung zur Detektion eines Lumineszenzereignisses in, an oder in der unmittelbaren Umgebung einer Zelle, eines Zellverbandes oder eines Gewebes, wobei in Figur 1 zu Zwecken der Veranschaulichung lediglich eine Zelle 6 dargestellt ist. Die Vorrichtung umfasst ein Trägerelement 1 mit einer für das direkte oder indirekte Ankoppeln von Zellen präparierten Oberfläche 100. Hierzu umfasst die Oberfläche beispielsweise eine Gelatineschicht, auf der die Zelle bereits gewachsen ist, oder eine andere für die Immobilisierung einer Zelle oder eines Zellverbandes geeignete Substanz. Oberhalb der Oberfläche 100 ist eine einen Hohlraum 70 bildende Abdeckung vorhanden, wobei die Zelle in diesem Hohlraum angeordnet ist. Die Abdeckung umfasst einen Zufluss 8 und einen Abfluss 9, wobei der Zufluss 8 an eine ein Anregungsmedium aufnehmende Anregungsquelle 21 angeschlossen ist.

Die Vorrichtung dient zur Untersuchung des Verhaltens der Zelle 6 bei einer chemischen oder biochemischen Anregung durch das in der Anregungsquelle 21 enthaltene Anregungsmedium mittels Lumineszenzmessungen. Die Lumineszenz wird durch Luminophore erzeugt, die in hinlänglicher bekannter Weise in die Zelle oder in eine Nährlösung in der Umgebung der Zelle eingebracht sind, und die beispielsweise mit einem Stoffwechselprodukt der Zelle reagieren. Die Lumineszenz wird durch Detektoren 2, beispielsweise Photodioden detektiert, die unterhalb der Oberfläche 100 in dem Trägerelement integriert sind. In dem Beispiel ist eine Wellenlängenfilter 4 zwischen der Oberfläche 100 mit der Zelle 6 und den Detektoren 2 ausgebildet, wobei auf den Detektoren 6 vorzugsweise eine nicht näher dargestellte optisch transparente Isolationsschicht aufgebracht ist, die Leckströme über die Oberfläche 100 verhindert, sofern das Filter 4 nicht elektrisch isolierend ist. zusätzlich mit einem Filter 4 versehen sein.

Auf den Träger 1 ist in dem Beispiel ein Kratzschutz 3 mit einer Oberflächenbeschichtung 5, beispielsweise einem Edelmetall oder einem hydrophoben/hydrophilen Material, aufgebracht, wobei dieser Kratzschutz 3 oberhalb der Detektoren eine Aussparung aufweist, am Boden derer das Filter 4 und die für die Aufnahme der Zelle präparierte Oberfläche 100 vorhanden ist.

In dem Trägerelement, beispielsweise einem Halbleiterchip, ist in dem Beispiel weiterhin eine - in der Figur 1 schematisch dargestellte - Auswerteschaltung 11 integriert, die an die Detektoren 2 angeschlossen ist. Leitungsverbindungen 21 zwischen der Auswerteschaltung 11 und den Detektoren 2 sind in Figur 1 lediglich schematisch dargestellt.

Dem Hohlraum 70 ist aus einem Flüssigkeitsreservoir 71 eine zum Aufbewahren bzw. Nasshalten oder auch Waschen der Zelle geeignete Flüssigkeit zuführbar, beispielsweise eine Nährlösung. Zur Anregung der Zelle wird dieser Flüssigkeit das Anregungsmedium aus der Anregungsquelle zugeführt, wobei die Zufuhr des Anregungsmediums in dem Beispiel mittels eines in der Zuführleitung 8 angeordneten Ventils 81 steuerbar ist. Die Steuerung dieses Ventils 81 erfolgt in dem Beispiel durch die integrierte Auswerteschaltung 11.

Das Anregungsmedium ist beispielsweise so gewählt, dass es den Stoffwechsel der Zelle beeinflusst, wobei Stoffwechselvorgänge durch die erläuterten Luminophore sichtbar und durch die Detektoren 2 detektiert werden. Vorzugsweise sind mehrere Anregungsquellen 21 vorhanden, die unabhängig voneinander ein Anregungsmedium abgeben, um so zeitlich aufeinanderfolgend das Zellenverhalten bei verschiedenen Anregungsmedien untersuchen zu können.

Um eine die Zelle 6 anregende chemische Substanz nach Ende der Untersuchung abzuführen wird beispielsweise mit der Flüssigkeit aus dem Reservoir 71 gespült, wodurch das Medium über den Abfluss 9 abfließt, oder die Flüssigkeit wird abgesaugt. Die Flüssigkeitszufuhr aus diesem Reservoir wird vorzugsweise ebenfalls über ein Ventil 72 gesteuert, das durch die Auswerteschaltung angesteuert ist.

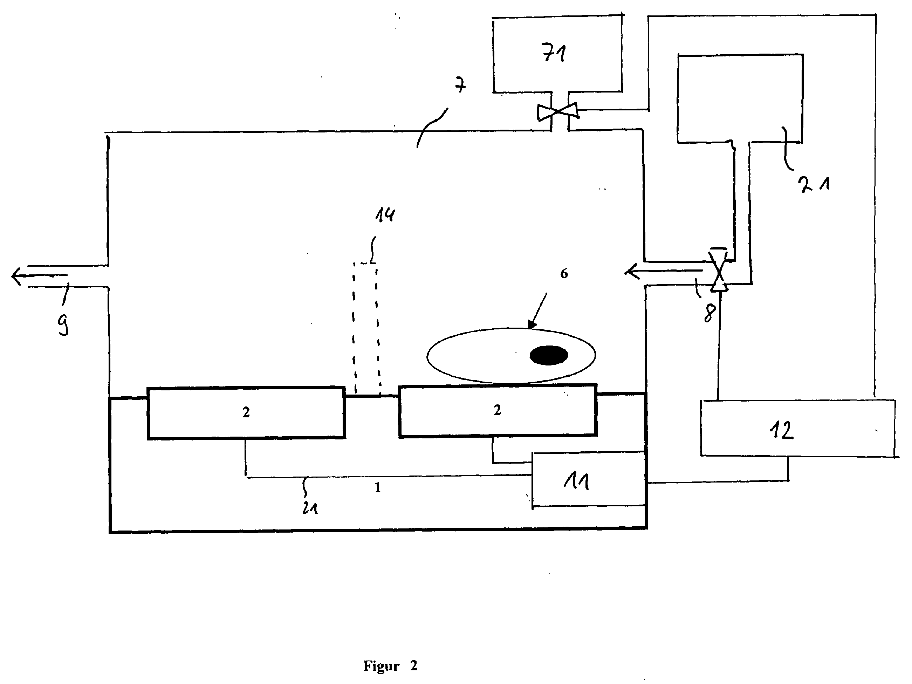

Die Steuerung der Ventile kann auch mittels einer externen Ansteuerschaltung 12 erfolgen, der auch ein von den Ausgangssignalen der Detektoren 2 abhängiges Signal der Auswerteschaltung 11 zugeführt sein kann, wie dies in Figur 2 dargestellt ist.

Figur 2 zeigt ein zweites Ausführungsbeispiel einer erfindungsgemäßen Vorrichtung, bei der zwei räumlich voneinander getrennte Detektoren 2 unterhalb einer für die Aufnahme von Zellen 6 geeigneten Oberfläche in einem Träger 1, beispielsweise einem, Halbleiterkörper 1 integriert sind. Dabei ist nur oberhalb eines der beiden Detektoren 2 eine Zelle 6 immobilisiert. Der andere Detektor dient zur Bereitstellung eines Referenzwertes für den durch den Detektor 2 unterhalb der Zelle 6 gelieferten Messwert. Der Referenzwert berücksichtigt dabei gegebenenfalls unabhängig von einer Anregung vorhandene, beispielsweise aus einer Eigenfluoreszenz der Systemkomponenten resultierende Lumineszenzsignale, die aus dem Messergebnis herauszurechnen sind. Wie gestrichelt dargestellt ist, ist vorzugsweise eine senkrecht von dem Träger 1 aufragende Trennwand 14 vorhanden, die verhindert, dass der den Referenzwert erzeugende Detektor 2 durch spezifische Lumineszenzsignale aus der Zelle 6 oder der Umgebung der Zelle beeinflusst wird.

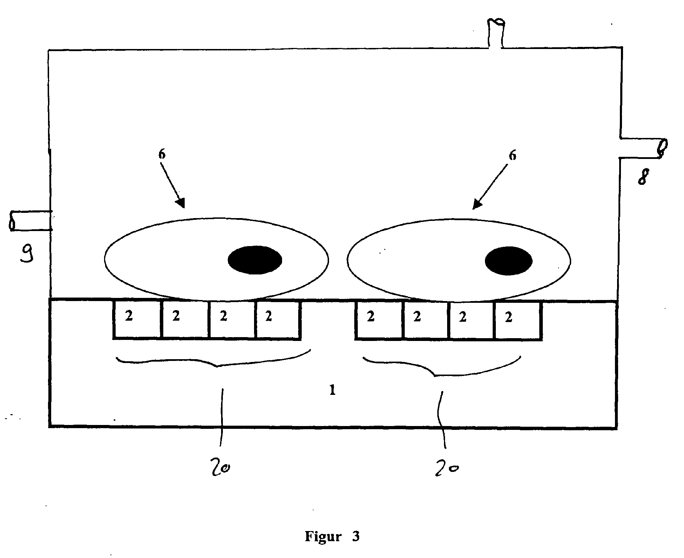

Figur 3 zeigt ein Ausführungsbeispiel einer erfindungsgemäßen Vorrichtung mit einer Trägeranordnung, die sich bezüglich der Anordnung der Detektoren 2 von den beiden vorherigen Ausführungsbeispielen unterscheidet. Auf die Darstellung der Anregungsquelle und der Auswerteschaltung die selbstverständlich auch vorhanden sind, ist bei dem Ausführungsbeispiel gemäß Figur 3 verzichtet.

Der vorzugsweise aus einem Halbleiterchip gebildete Träger umfasst in dem Beispiel zwei Detektorfelder 20, die jeweils mehrere Detektoren umfassen. Hierdurch können mehrere Messungen gleichzeitig durchgeführt werden, wodurch eine statistische Abschätzung des spezifischen erhaltenen Messsignals abgeleitet werden kann. Beispielsweise wird hierdurch ermöglicht, dass zwischen unspezifischen und spezifischen Signalen differenziert werden kann, wobei spezifische Signale die aus Stoffwechselvorgängen in der Zelle resultierenden Signal und unspezifische Signale andere Signale, beispielsweise Störsignale, sind. Diese unspezifischen Signale besitzen eine andere Verteilung als die spezifischen Signale, so dass eine Unterscheidung möglich wird.

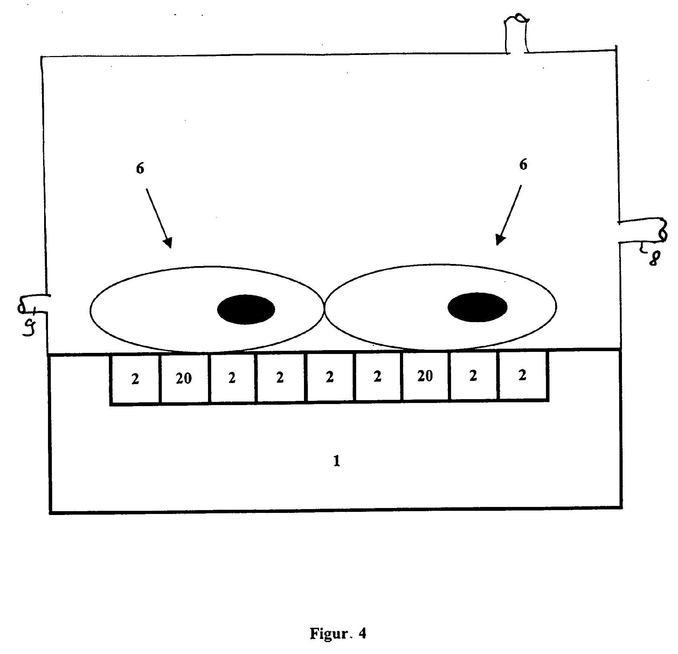

Figur 4 zeigt eine Abwandlung eines in Figur 3 dargestellten Trägerelements 2, das ein zusammenhängendes Detektorfeld 20 mit einer Vielzahl von Detektoren 2 aufweist, welches dazu geeignet ist mehrere Zellen gleichzeitig zu untersuchen. Diese Vorrichtung ermöglicht es, identische Zellen unter vergleichbaren Bedingungen zu untersuchen und deren Signale zu detektieren, wobei diese unter gleichen Bedingungen gewonnenen Signale zur statistischen Absicherung in einer Auswerteeinheit gemittelt werden können.

Ein Verfahren zur Herstellung eines für die erfindungsgemäße Vorrichtung geeigneten Trägers wird nachfolgend kurz erläutert:

Ein Halbleiterchip wird unter Verwendung von 6"(Inch) Wafern mit einem 0.5 µm CMOS-Prozess gefertigt. Die Detektoren 2 werden als pn-Photodioden in einer n-Wanne auf einem p-Substrat angeordnet. Nach der Feldoxidation folgen die Definition der p-Gebiete der Photodiode und die Aufbringung einer 10 nm dicken Gateoxidschicht. Gewünschtenfalls kann an dieser Stelle zusätzlich noch ein strukturiertes Nitrid (z.B. LPCVD oder PECVD) als UV -Filter aufgebracht werden. Dann erfolgt die Auflagerung und Strukturierung einer Siliziumdioxid-Schicht. Anschließend werden die weiteren üblichen CMOS-Schritte durchgeführt, wie z.B. das Aufbringen einer Verdrahtungsschicht und die Oberflächenpassivierung (Kratzschutz).

Der so hergestellte CMOS-Sensor wird durch Beschichten mit einem geeigneten Zellwachstumssubstrat, z.B. Gelatine, modifiziert. Auf dieses Substrat werden dann vereinzelte Zellen (z.B. trypsinisierte Epithelzellen) ausgesät und mit Zellkulturmedium (z.B. DMEM-FI2) zum Wachsen gebracht.

Die erfindungsgemäße Vorrichtung eignet sich beispielsweise zur Untersuchung des Kalziumstoffwechsels von Herzmuskelzellen. Dabei werden die Zellen zunächst durch osmotische Schockbehandlung mit Aqueorin beladen. Intrazelluläre Kalziumsignale als Antwort auf chemische Reize durch die aus der Anregungsquelle 21 abgegebene chemische Substanz werden durch die Biolumineszenz des Aqueorins sichtbar und durch die Detektoren detektiert.

Die erfindungsgemäße Vorrichtung zur Untersuchung intrazellulärer Vorgänge nach einer biologischen oder chemischen Anregung ist besonders kompakt und mittels bekannter Verfahren herstellbar.

## Patentansprüche

1. Vorrichtung zur Detektion eines Lumineszenzereignisses in, an oder in der unmittelbaren Umgebung einer Zelle, eines Zellverbandes oder eines Gewebes, die folgende Merkmale aufweist:
(a) ein Trägerelement (1) mit einer für das direkte oder indirekte Ankoppeln von Zellen präparierten Oberfläche (100),
(b) wenigstens einen optischen Detektor (2) zum Empfangen eines Lumineszenzsignals, der in dem Trägerelement (1) unterhalb der Oberfläche (100) integriert ist,
**gekennzeichnet, durch** folgende weitere Merkmale:
(c) eine die Oberfläche (100) unter Bildung eines Hohlraumes (70) überdeckende Abdeckung (7), die eine Zuflussöffnung (8) und eine Abflussöffnung (9) aufweist,
(d) eine an die Zuflussöffnung (8) angeschlossene Anregungsquelle (21).

2. Vorrichtung nach Anspruch 1, bei der ein Filter (4) zwischen der Oberfläche (100) und dem wenigstens einen Detektor (2) ausgebildet ist.

3. Vorrichtung nach Anspruch 1 oder 2, bei der das Trägerelement (1) ein Halbleiterkörper ist.

4. Vorrichtung nach einem der vorangehenden Ansprüche, bei der mehrere Detektoren (2) unterhalb der für das Ankoppeln der Zellen präparierten Oberfläche (100) in dem Trägerelement (1) integriert sind.

5. Vorrichtung nach Anspruch 4, bei der der wenigstens ein Detektor (2) eine Photodiode ist.

6. Vorrichtung nach einem der vorangehenden Ansprüche, bei der eine an den wenigstens einen Detektor (2) angeschlossene Auswerteschaltung (11) vorgesehen ist.

7. Vorrichtung nach Anspruch 6, bei der die Auswerteschaltung (11) in dem Trägerelement (1) integriert ist.

8. Vorrichtung nach Anspruch 6 oder 7, bei der die Anregungsquelle (21) angesteuert durch die Auswerteschaltung eine chemische oder biologische Substanz an die Zuflussöffnung (8) abgibt.

9. Vorrichtung nach Anspruch 8, bei der zur Steuerung der Medienzufuhr ein Ventil in einer Zuführleitung zwischen der Anregungsquelle (21) und der Zuflussöffnung (8) angeordnet ist.

10. Vorrichtung nach einem der vorangehenden Ansprüche, bei der eine Adhäsionsmatrix und/oder ein Wachstumssubstrat für Zellen auf die Oberfläche (100) aufgebracht ist.

11. Vorrichtung nach Anspruch 10, bei der das Wachstumssubstrat Gelatine ist.

12. Vorrichtung nach einem der Ansprüche 1 bis 9, bei der ein Zellen immobilsierendes Medium auf die Oberfläche (100) aufgebracht ist.

13. Vorrichtung nach Anspruch 12, bei der das Medium Polystyrol, vorzugsweise negativ geladenes Polystyrol ist.

14. Vorrichtung nach einem der vorangehenden Ansprüche, bei der wenigstens eine Zelle (6) an der Oberfläche immobilisiert ist.

15. Vorrichtung nach einem der vorangehenden Ansprüche, bei der die für die Aufnahme von Zellen präparierte Oberfläche der Trägervorrichtung gegenüber Oberflächenbereichen (101), die nicht für die Aufnahme von Zellen präpariert sind, vertieft ausgebildet ist, wobei diese Vertiefung vorzugsweise wenigstens 100nm beträgt.

16. Verfahren zur Detektion eines Lumineszenzereignisses in, an oder in der unmittelbaren Umgebung einer Zelle, eines Zellverbandes oder eines Gewebes, das folgende Merkmale aufweist:
- Bereitstellen einer Sensorvorrichtung nach einem der Ansprüche 1 bis 16,
- Immobilisieren der Zelle an der für die Aufnahme von Zellen präparierten Oberfläche (100),
- Einbringen eines mit einem Stoffwechselprodukt der Zelle reagierendes Luminophors in die Zelle (6) oder in die Umgebung der Zelle,
- Anregen der Zelle mittels einer chemischen oder biologischen Substanz,
- Detektieren eines Lumineszenzsignals.

17. Verfahren nach Anspruch 15 oder 16, bei dem das Lumineszenzsignal zeitlich aufgelöst detektiert wird.
